# NEUE EUROPÄISCHE PATENTSCHRIFT

(11) **EP 3 576 825 B2**
(45) Veröffentlichungstag und Bekanntmachung des Hinweises auf die Entscheidung über den Einspruch: **01.04.2026**
(45) Hinweis auf die Patenterteilung: 16.12.2020
(21) Anmeldenummer: 17787330.4
(22) Anmeldetag: 20.10.2017
(51) Int. Cl.: A61M 15/08, A61F 9/00, B65D 47/20

(54) **AUSTRAGVORRICHTUNG FÜR EIN FLÜSSIGES MEDIUM**
DISCHARGE DEVICE FOR A LIQUID MEDIUM
DISPOSITIF DE DISTRIBUTION POUR UN MILIEU FLUIDE

(30) Priorität: 01.02.2017 US 201762453256 P
(43) Veröffentlichungstag der Anmeldung: 11.12.2019
(73) Patentinhaber: Silgan Dispensing Systems Hemer GmbH, 58675 Hemer (DE)
(72) Erfinder: HÖHM, Sven-Uwe, 58091 Hagen (DE); HARMS, Heiko, 58708 Menden (DE)
(74) Vertreter: Henseler, Daniela
(86) Internationale Anmeldenummer: PCT/EP2017/001235
(87) Internationale Veröffentlichungsnummer: WO 2018/141350

(56) Entgegenhaltungen:
- WO-A1-2014/048668
- DE-A1- 102004 050 679
- DE-A1- 102004 050 977
- DE-A1- 102006 008 874
- DE-A1- 102009 006 430
- DE-A1- 102009 006 431
- DE-A1- 102010 063 592
- DE-A1- 102014 201 697

## Beschreibung

Die Erfindung betrifft eine Austragvorrichtung für ein flüssiges Medium nach dem Oberbegriff des Anspruchs 1.

Aus DE 10 2010 063 592 A1 ist ein derartiger Spender für eine Flüssigkeit bekannt. Der Spender dient insbesondere pharmazeutischen Zwecken und wird zur Ausgabe von diesbezüglichen Flüssigkeiten verwendet, und zwar für eine Applikation dieser Flüssigkeiten in den Mund, die Nase, die Augen oder die Ohren eines Patienten. Der Spender verfügt über einen flaschenförmigen Flüssigkeitsspeicher sowie über eine Auslassbaugruppe. Die Auslassbaugruppe endet an ihrem dem Flüssigkeitsspeicher abgewandten Ende in einer Austragöffnung, die in einem Schließzustand des Spenders durch ein Ventil verschlossen wird. Das Ventil verfügt hierzu über einen im Bereich der Austragöffnung innenseitig angeordneten Ventilsitz sowie einen gegenüber dem Ventilsitz beweglichen Ventilkörper. Der Ventilkörper ist mittels einer Ventilfeder kraftbeaufschlagt, so dass dieser in einem Ruhezustand am Ventilsitz derart anliegt, dass die Austragöffnung von einer Ventilvorkammer getrennt ist. Die Ventilvorkammer wird außenseitig durch ein Außenbauteil der Auslassbaugruppe begrenzt. Innenseitig wird die Ventilvorkammer durch eine Druckbeaufschlagungsfläche des Ventilkörpers begrenzt. Sobald der Flüssigkeitsdruck in der Ventilvorkammer über einen durch die Größe der Druckbeaufschlagungsfläche und der Ausgestaltung der Feder bestimmten Grenzdruck steigt, bewirkt dieser Druck eine partielle Verlagerung des Ventilkörpers und Abheben des Ventilkörpers vom Ventilsitz. Lediglich ein außenseitiger Einspannbereich ist hierbei lageunveränderlich. Durch die Verlagerung insbesondere eines Schließstiftes des Ventilkörpers gegenüber dem Ventilsitz wird das Ausströmen von Flüssigkeit durch die Austragöffnung möglich.

Beim vorbekannten Spender ist die Ventilfeder vergleichsweise schwach ausgebildet. Sie drückt den Ventilkörper mit einer Kraft von vorzugsweise zwischen 2 N und 3 N gegen den Ventilsitz. Verbunden mit einer vergleichsweise großen Druckbeaufschlagungsfläche führt dies dazu, dass die Auslassöffnung bereits bei einem vergleichsweise geringen Überdruck in der Ventilvorkammer geöffnet wird, vorliegend bei 0,3 bar. Die Flüssigkeit aus der Ventilvorkammer strömt daher in nur gering druckbeaufschlagtem Zustand durch die Austragöffnung, was der bestimmungsgemäßen Erzeugung eines Tropfens an der Auslassseite der Austragöffnung dient. Beim bekannten Spender ist vorgesehen, dass die Druckerzeugung in der Ventilvorkammer dadurch erfolgt, dass der als Flasche ausgebildete Flüssigkeitsspeicher manuell zusammengedrückt wird. Der sich dadurch erhöhende Druck im Flüssigkeitsspeicher setzt sich über einen Flüssigkeitspfad bis zur Ventilkammer fort.

Gerade bei solchen Spendern besteht allerdings das Problem der Verkeimung im Bereich der Ventilflächen an der Auslassöffnung. Der geringe Öffnungsdruck, der zur Erzeugung von Tropfen dient und der verhindern soll, dass es zur ungewollten Ausbringung eines Sprühstrahls kommt, hat zur Folge, dass die Ventilflächenpressung nahe der Auslassöffnung des Spenders im Schließzustand nicht ausreichend groß ist, um nach einem Austragvorgang das Eintreten von Verunreinigungen in den Innenbereich des Spenders zuverlässig zu verhindern. Da aber der bestimmungsgemäße Zweck dieser Spender üblicherweise ist, dass die auszutragende Flüssigkeit auf dem Körper aufgebracht oder in den Körper eingebracht wird, um möglichst schnell vom Körper aufgenommen zu werden, ist die Gefahr groß, dass Verunreinigungen in die Flüssigkeit gelangen und dort zu einer Keimbelastung führen, die Erkrankungen des Patienten zur Folge haben kann. Bekannt ist deshalb, dass mindestens eine Ventilfläche des Auslassventils füssigkeitsabweisend, keimtötend und/oder Keimwachstum verhindernd ausgebildet ist. Das Fortschreiten der Kontamination in den Spender hinein soll dadurch verhindert werden. Als nachteilig erwiesen hat sich jedoch der Zwang zur Verwendung von keimtötenden bzw. Keimwachstum verhindernden Ausgestaltungen von Ventilflächen des Auslassventils.

Aufgabe der Erfindung ist es daher, eine Austragvorrichtung nach dem Oberbegriff des Anspruchs 1 zu schaffen, die für die Abgabe von Tropfen ausgelegt ist und dabei eine von außen eintretende Verunreinigung von Flüssigkeit zuverlässig verhindert.

Diese Aufgabe wird durch die Merkmale des Anspruchs 1 gelöst.

Hierdurch wird eine Austragvorrichtung geschaffen, die sofort schließt, sobald der Flüssigkeitsdruck in der Ventilvorkammer nicht mehr ausreicht, um einen Öffnungszustand und ein Ausströmen von Flüssigkeit zu bewirken, wobei der Öffnungsdruck durch die Art der außenseitigen Einspannung der Druckbeaufschlagungsfläche des Ventilkörpers feinfühlig einstellbar ist. Ein gesamter Membrandurchmesser der Druckbeaufschlagungsfläche kann so für die Ventilöffnung zur Verfügung gestellt werden.

Zudem kann innenseitig benachbart des erfindungsgemäßen Einspannbereiches der Druckbeaufschlagungsfläche eine flächenhafte Unterlage als partieller Stützring für eine Membran, an der die Druckbeaufschlagungsfläche ausbildbar ist, am Austragkopf vorgesehen sein. Verformt sich die Membran für einen Medienhub, dann kann nach einem anfänglichen Verformungsweg der Membran diese sich randseitig auf die Unterlage aufsetzen. Der effektive Durchmesser der Druckbeaufschlagungsfläche wird entsprechend der radialen Breite der Unterlage verkleinert und die einwirkende Kraft zur Membranverformung verkleinert, weil ein Teil der einwirkenden Kraft in den Stützring der Unterlage eingeleitet wird. Es kommt dann folglich zu einer Bewegungswiderstandserhöhung, während sich die Ventilvorkammer für einen Medienhub füllt. Die Anlage der Membran an der Unterlage kann als taktil erfassbarer Kraftveränderungspunkt längs eines Verformungsweges der Membran für beispielsweise eine Tropfenabgabe genutzt werden. Dies verbessert die Kontrollierbarkeit der Tropfenabgabe und kann so ein Überströmen (Strahlbildung) vermindern. Das haptische Ereignis eines anschlagartigen Druck-Hub-Widerstandes kann an den Anwender eine zusätzlich fühlbare Rückmeldung liefern, wann eine Tropfenabgabe erfolgen wird. Eine graduelle Erhöhung der Widerstandskraft kann erfasst werden.

Das Kraft/Weg-Verhalten (taktile Rückmeldung) der Membranverformung kann für verschiedene Anwendungsfunktionen durch Variieren der Betätigungskräfte eingesetzt werden. So kann die radiale Breite und Höhe der Unterlage verschieden gewählt werden, um das Maß der Verringerung des effektiven Membrandurchmessers einstellen zu können. Auch kann die Unterlage umfänglich asymmetrisch ausgeformt sein, wodurch sich ein Verkippen des Ventilstößels zur Austragöffnung ergeben kann. Das an der Austragöffnung auszugebende Medium, insbesondere Tropfen, kann quasi abgeschert werden, da ein Durchgang für das Medium nur partiell freigegeben wird. Möglichen Kohäsionskräften, die eine Art Ankleben des Tropfens bei der Tropfenabgabe bewirken, kann entgegengewirkt werden. Besonders vorteilhaft ist, dass die Austragvorrichtung für ein fluides Medium dabei leicht und zuverlässig betätigbar und einfach handhabbar bleibt.

Ist die Austragvorrichtung insbesondere für einen Überkopfbetrieb vorgesehen, kann ferner vorgesehen sein, dass ein Belüftungswegausgang eines Druckausgleichskanals in den Vorratsbehälter über eine am Austragkopf vorgesehene Dichtvorrichtung rückschlagventilartig verschließbar ist. Die Dichtvorrichtung kann beispielsweise eine konkav zum Inneren des Vorratsbehälters gebogene Dichtscheibe sein, deren Scheibenrand dichtend anliegt. Der Scheibenrand kann auch mit Dichtlippen versehen sein, die der Dichtscheibe ein korkenartiges Anlegeverhalten an eine Wandung eines Belüftungswegausgangs unter Ausbilden eines Dichtstopfens verleihen.

Weitere Ausgestaltungen und Vorteile der Erfindung sind der nachfolgenden Beschreibung und den Unteransprüchen zu entnehmen.

Die Erfindung wird nachstehend anhand der in den beigefügten Abbildungen dargestellten Ausführungsbeispiele näher erläutert.
Fig. 1 zeigt schematisch eine perspektivische Ansicht einer Austragvorrichtung mit einem Vorratsbehälter und mit einem einen Tropfadapter aufweisenden Austragkopf,
Fig. 2 zeigt schematisch eine perspektivische Ansicht des Austragkopfes gemäß Fig. 1 in vergrößerter Darstellung und unter Weglassung des Tropfadapters,
Fig. 3 zeigt schematisch einen Längsschnitt des Austragkopfes gemäß einem ersten Ausführungsbeispiel mit Darstellung eines Flüssigkeitsweges, wobei der Austragkopf an einem Hals eines Vorratsbehälters befestigt ist,
Fig. 4 zeigt schematisch einen Teilbereich des Austragkopfes gemäß Fig. 3 mit Darstellung einer Membranverformung unter Mediendruck bei Betätigung,
Fig. 5 zeigt schematisch einen weiteren Längsschnitt des Austragkopfes gemäß dem ersten Ausführungsbeispiel mit Darstellung des Flüssigkeitsweges und einer Membranverformung ohne Kontakt mit einem Tellerrand,
Fig. 6 zeigt schematisch einen Längsschnitt des Austragkopfes gemäß einem zweiten Ausführungsbeispiel mit Darstellung des Flüssigkeitsweges und einer Membranverformung mit Verkleinerung einer Druckbeaufschlagungsfläche durch Anschlagen der Membran auf den Tellerrand durch Tellerranderhöhung,
Fig. 7 zeigt schematisch einen Längsschnitt des Austragkopfes gemäß einem dritten Ausführungsbeispiel mit Darstellung des Flüssigkeitsweges und einer Membranverformung mit Verkleinerung einer Druckbeaufschlagungsfläche durch Anschlagen der Membran auf den Tellerrand durch Tellerranderhöhung und Tellerrandverbreiterung ,
Fig. 8 zeigt schematisch einen Längsschnitt des Austragkopfes gemäß einem vierten Ausführungsbeispiel mit Darstellung des Flüssigkeitsweges und einer Membranverformung mit Verkleinerung einer Druckbeaufschlagungsfläche durch Anschlagen der Membran auf den Tellerrand durch Tellerranderhöhung und asymmetrischer umfänglicher Tellerrandverbreiterung,
Fig. 9a zeigt schematisch im Schnitt die Ausgestaltung eines Ventilsitzes in der Austragöffnung mit flächiger Abdichtung in geschlossenem Zustand,
Fig. 9b zeigt schematisch im Schnitt die Ausgestaltung des Ventilsitzes in der Austragöffnung mit flächiger Abdichtung in geöffnetem Zustand,
Fig. 10a zeigt schematisch im Schnitt die Ausgestaltung eines Ventilsitzes in der Austragöffnung mit konischer Abdichtung in geschlossenem Zustand,
Fig. 10b zeigt schematisch im Schnitt die Ausgestaltung des Ventilsitzes in der Austragöffnung mit konischer Abdichtung in geöffnetem Zustand,
Fig. 11 zeigt schematisch einen Längsschnitt des Austragkopfes gemäß dem ersten Ausführungsbeispiel nach Fig. 3 mit Darstellung eines Belüftungsweges, wobei eine Dichtscheibe als Dichtvorrichtung an einem Belüftungswegausgang in den Vorratsbehälter vorgesehen ist,
Fig. 12 zeigt schematisch einen Längsschnitt des Austragkopfes gemäß dem ersten Ausführungsbeispiel nach Fig. 3 mit Darstellung eines Belüftungsweges, wobei ein Dichtstopfen als Dichtvorrichtung an einem Belüftungswegausgang in den Vorratsbehälter vorgesehen ist.

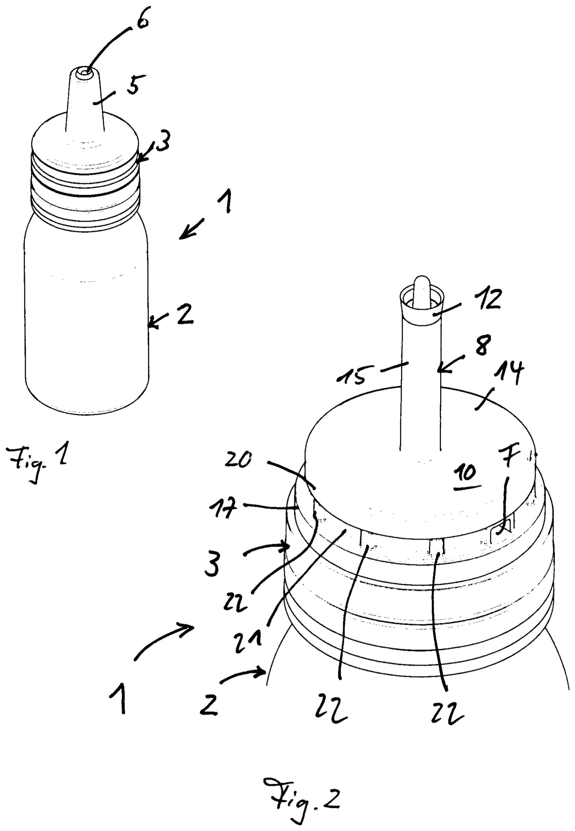

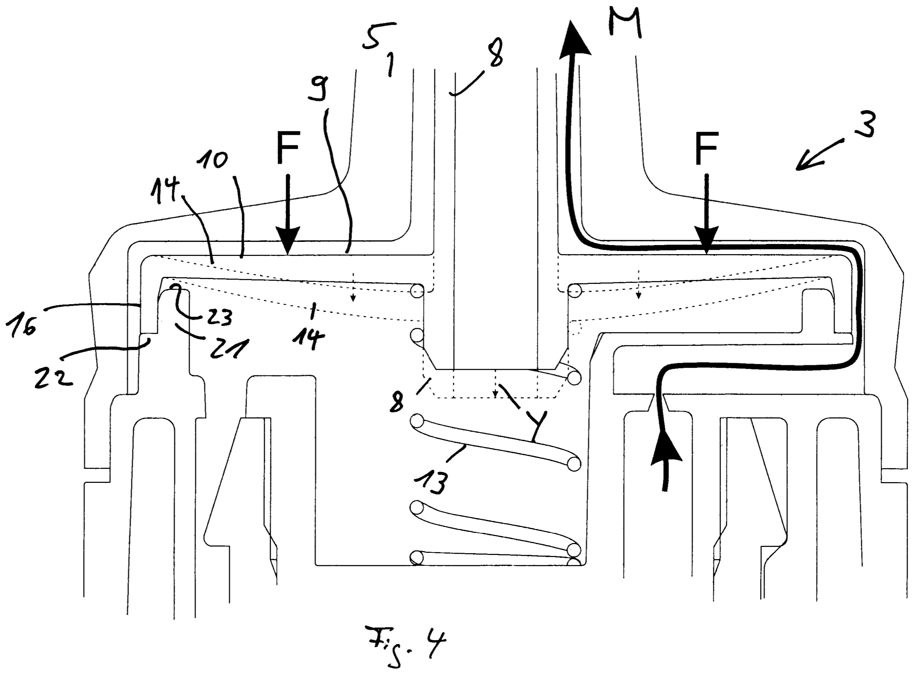

Fig. 1 zeigt eine Austragvorrichtung 1 für ein flüssiges Medium. Diese Austragvorrichtung 1 verfügt über einen Vorratsbehälter 2 zur Aufnahme von Medium und einen an dem Vorratsbehälter 2 festlegbaren Austragkopf 3. Der Vorratsbehälter 2 ist vorzugsweise flaschenförmig ausgebildet und manuell zusammendrückbar, so dass der sich dadurch erhöhende Druck im Vorratsbehälter 2 einen Mediumhub verursacht. Der Austragkopf 3 ist vorzugsweise über einen Verschluss 4 (vgl. Fig. 4), beispielsweise einen Schnappverschluss, an einem Hals des Vorratsbehälters 2 befestigt. Die Austragvorrichtung 1 ist gemäß dargestelltem Ausführungsbeispiel als Tropfer ausgebildet, beispielsweise für die Ausgabe von Augentropfen.

Der Austragkopf 3 umfasst dazu als ein Außenbauteil einen Abgabeadapter, der hier ein Tropfadapter 5 ist. Der Austragkopf 3 weist eine Austragöffnung 6 zum Austragen von Medium aus dem Vorratsbehälter 2 auf, in der der vorzugsweise vorgesehene Tropfadapter 5 an seinem dem Vorratsbehälter 2 abgewandten Ende endet. Die Austragöffnung 6 ist in einem Schließzustand durch ein Auslassventil 7 verschließbar. Der hier als Tropfadapter 5 vorgesehene Abgabeadapter ist mit einem tubusförmigen Fortsatz ausgebildet (Fig. 1).

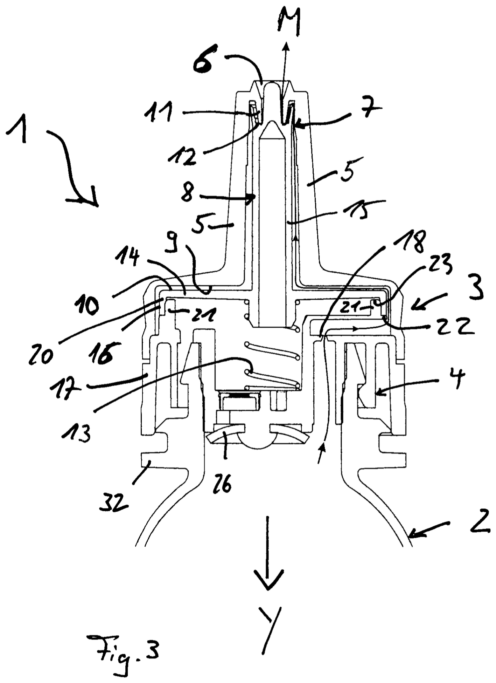

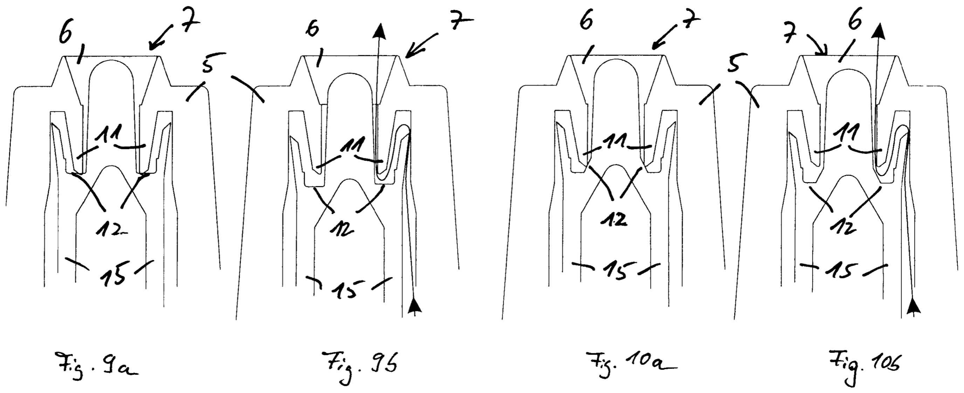

Wie Fig. 2 und Fig. 3 zeigen, verfügt das der Austragöffnung 6 zugeordnete Auslassventil 7 über einen vorgespannten, insbesondere federvorgespannten Ventilkörper 8, der eine Ventilvorkammer 9 durch eine Druckbeaufschlagungsfläche 10 begrenzt. Im Bereich der Austragöffnung 6 weist das Auslassventil 7 einen innenseitig angeordneten Ventilsitz 11 (vgl. Fig. 9a, 9b, 10a, 10b) auf, gegenüber dem der Ventilkörper 8 axial beweglich ist. Der Ventilkörper 8 kann mittels einer Ventilfeder 13, insbesondere einer Druckfeder, in Richtung der Austragöffnung 6 kraftbeaufschlagt werden, so dass dieser in einem Schließzustand am Ventilsitz 11 mit einer Dichtkante 12 derart anliegt, dass die Austragöffnung 6 von der Ventilvorkammer 9 getrennt ist. Die Ventilvorkammer 9 wird außenseitig durch den Tropfadapter 5 begrenzt. Innenseitig wird die Ventilvorkammer 9 durch die Druckbeaufschlagungsfläche 10 des Ventilkörpers 8 begrenzt. Der Ventilkörper 8 bildet die Druckbeaufschlagungsfläche 10 an einer Membran 14 aus, von der sich ein Ventilstößel 15 erhebt, der kopfseitig die Dichtkante 12 oder ein sonstiges Dichtelement 12 trägt. Membran 14 und Ventilstößel 15 sind vorzugsweise einstückig geformt.

Die Druckbeaufschlagungsfläche 10 weist außenseitig einen lageunveränderlichen Einspannbereich 16 auf. Die Membran 14 ist dazu randseitig an einem Innenbauteil 17 des Austragkopfes 3 fluiddicht befestigt, und zwar laserverschweißt. Das Innenbauteil 17 trägt ferner den Verschluss 4 zur Befestigung am Vorratsbehälter 2.

Sobald der Flüssigkeitsdruck in der Ventilvorkammer 9 über einen durch die Größe der Druckbeaufschlagungsfläche 10 und der Ausgestaltung der Ventilfeder 13 bestimmten Grenzdruck steigt, bewirkt dieser Druck eine partielle Verlagerung des Ventilkörpers 8 in Richtung Y, d.h. die Dichtkante 12 hebt vom Ventilsitz 11 ab durch eine Bewegung des Ventilkörpers 8 weg von der Austragöffnung 6. Durch diese Verlagerung des Ventilkörpers 8 wird das Ausströmen von Flüssigkeit durch die Austragöffnung 6 möglich. Ein zugehöriger Flüssigkeitsweg bzw. Medienpfad M aus dem Vorratsbehälter 2 durch den Austragkopf 3 und dort durch die Ventilvorkammer 9 und das Auslassventil 7 ist in Fig. 3 angegeben. Beim Austritt aus dem Vorratsbehälter 2 ist vorzugsweise eine Fließbremse 18 vorgesehen. Hierbei handelt es sich um eine enge Durchgangsstelle, durch die das Medium zur Austragöffnung 6 hindurchfließen muss und wodurch eine Drosselwirkung erzielt wird. Der Strömungswiderstand der Fließbremse 18 verhindert ein Überströmen des Medienpfades F und bewirkt einen Druckabfall, was für die Ausgabe von Tropfen vorteilhaft ist, wenn der Vorratsbehälter 2 manuell quer zur Axialrichtung zusammengedrückt wird und Medium entlang des Medienpfads M in die Ventilvorkammer 9 gedrückt wird.

Bei der Austragvorrichtung 1 gemäß Fig. 1 bis Fig. 3 ist die Ventilfeder 13 nämlich vergleichsweise schwach ausgebildet. Verbunden mit einer vergleichsweise großen flachen Druckbeaufschlagungsfläche 10 führt dies dazu, dass die Austragöffnung 6 bei einem vergleichsweise geringen Überdruck von beispielsweise 0,2 bis 0,5 bar in der Ventilvorkammer 9 geöffnet wird. Das Medium strömt dann in nur gering druckbeaufschlagtem Zustand durch die Austragöffnung 6, was der vorgesehenen Erzeugung eines Tropfens an der Austragöffnung 6 dient.

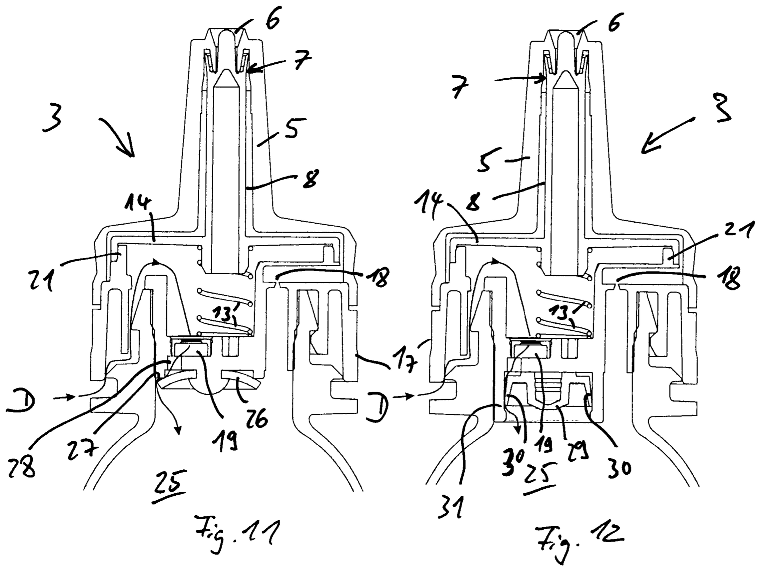

Die Austragvorrichtung 1 weist ferner einen in den Vorratsbehälter 2 mündenden Druckausgleichskanal D mit einer darin eingesetzten mikrobiologisch wirksamen Filteranordnung 19 auf, wie nachfolgend noch in Zusammenhang mit Fig. 11 und Fig. 12 beschrieben wird. Der Druckausgleichskanal D ist von dem Medienpfad M getrennt.

Fig. 2 bis Fig. 4 zeigen weiterhin, dass die Druckbeaufschlagungsfläche 10 als Einspannbereich 16 einen kappenartigen Randfortsatz 20 aufweist, der innenseitig auf einen Tellerrand 21 des Innenbauteils 17 des Austragkopfes 3 aufsetzbar ist. Der Tellerrand 21 weist dazu einen sich radial nach außen erstreckenden Anschlag 22 auf, der eine Aufstandsfläche für den Randfortsatz 20 bildet unter Beabstandung der Druckbeaufschlagungsfläche 10 von einer Kopffläche 23 des Tellerrandes 21. Wie Fig. 2 zeigt, kann der Anschlag 22 von einzelnen umfänglich verteilt und zueinander beabstandet angeordneten Teilanschlägen gebildet werden, die dann zusammen den Anschlag 22 bilden. Der Tellerrand 21 ist ein um eine axiale Mittelachse der Austragvorrichtung 1 am Innenbauteil 17 angeformter ringförmiger Steg, der bezüglich Höhe und/oder Breite variiert werden kann.

Wie insbesondere in Fig. 4 dargestellt, kann der Einspannbereich 16 über die Membran 14 derart ausgebildet sein, dass bei einer Membranverformung unter Mediendruck bei Betätigung (gestrichelt dargestellt) keine Abstützung der Membran 14 am Tellerrand 21 eintritt. Als Druckbeaufschlagungsfläche 10 ist deshalb der gesamte Flächeninhalt wirksam. Eine Maximierung der Größe der wirksamen Druckbeaufschlagungsfläche 10 ist durch den erfindungsgemäßen Einspannbereich 16 für jeden Flächeninhalt einer Druckbeaufschlagungsfläche 10 möglich.

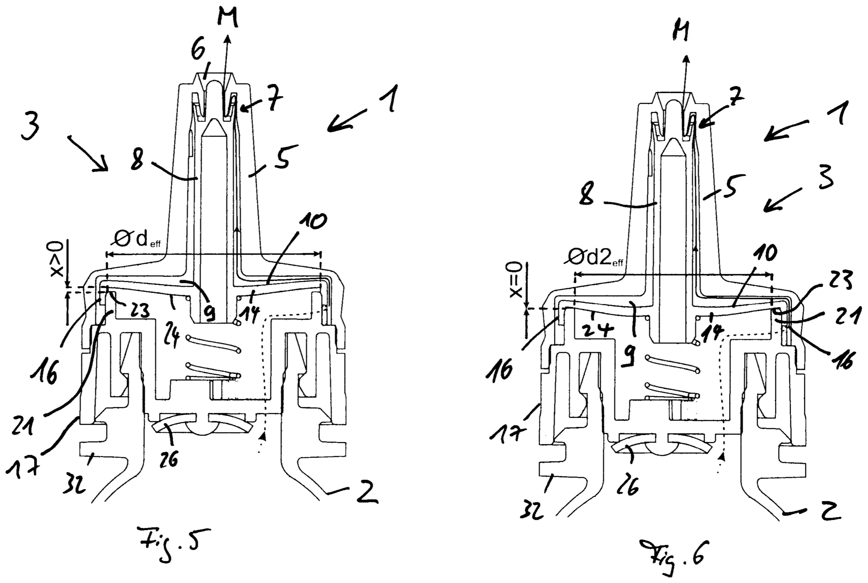

Eine Membranverformung ohne Kontakt mit dem Tellerrand 21 ist auch in Fig. 5 dargestellt. Dort ist ein Abstand x zwischen der Kopffläche 23 des Tellerrandes 21 und einer Unterseite 24 der Membran 14 mit x > 0 angegeben. Ferner angegeben ist der effektive Durchmesser der Druckbeaufschlagungsfläche 10 bei Einsatz des erfindungsgemäßen Einspannbereiches 16 der Membran 14. Dieser Abstand x ist angegeben für den Grenzdruck in der Ventilvorkammer 9, der zu einer partiellen Verlagerung des Ventilkörpers 8 führt und bestimmt ist durch die Größe der Druckbeaufschlagungsfläche 10 und die Ausgestaltung der Ventilfeder 13.

Der Tellerrand 21 bildet somit mit der Kopffläche 23 eine flächenhafte Unterlage für eine Abstützung, vorzugsweise partielle randseitige Abstützung der Membran 14 bei einer Membranverformung unter Mediendruck. Die Membran 14 ist dazu elastisch flexibel zum Verändern eines Kammervolumens der Ventilvorkammer 9.

Fig. 6 zeigt ein zweites Ausführungsbeispiel, bei dem der Tellerrand 21 von einem Ringsteg gebildet wird, der höher ausgebildet ist als der im ersten Ausführungsbeispiel gemäß Fig. 5. Die Höhe ist derart gewählt, dass sich die Membran 14 auf der Kopffläche 23 des Tellerrandes 21 vor oder bei Erreichen des Grenzdrucks in der Ventilvorkammer 9 durch Verändern des Kammervolumens abstützt. Der Abstand x ist dann 0, und der effektive Durchmesser der Druckbeaufschlagungsfläche 10 bei Einsatz des erfindungsgemäßen Einspannbereiches 16 der Membran 14 ist verringert durch das Maß der Durchmesserbreite des Tellerrandes 21. Soweit die Membran 14 sich auf der Kopffläche 23 des Tellerrandes 21 abstützt, wird die vom Medium in der Ventilvorkammer 9 ausgeübte Kraft F in den Tellerrand eingeleitet und steht nicht als effektive Druckbeaufschlagungsfläche 10 zur Verfügung. Eine Verkleinerung der Druckbeaufschlagungsfläche 10 hat zur Folge, dass eine höhere Kraft F aufgewendet werden muss, um eine weitere Membranverformung zu erreichen, d.h. mehr Flüssigkeit in die Ventilvorkammer 9 einbringen zu können, die zum Erreichen des Grenzdrucks erforderlich ist. Dieses Kraft/Weg-Verhalten bei der Auslenkung der Membran 14 kann als taktile Rückmeldung bei einer Tropfenabgabe genutzt werden, insbesondere dann, wenn eine Verkleinerung der effektiven Druckbeaufschlagungsfläche 10 vor Erreichen des Grenzdrucks über die Höhe des Tellerrandes 21 bestimmt wird. Dem Anwender kann auf diese Weise das Erreichen des Grenzdrucks und damit die Abgabe eines Tropfens durch Öffnen der Austragöffnung 6 über ein haptisches Ereignis angezeigt werden, dass nämlich das Bevorstehen des Erreichens des Grenzdrucks durch erhöhte Druckerzeugung in der Ventilvorkammer 9 und damit als Bewegungswiderstandserhöhung beim Zusammendrücken des Vorratsbehälters 2 fühlbar gemacht werden kann. Erfindungsgemäß kann quasi ein Druckpunkt bei der Tropfenabgabe eingestellt werden.

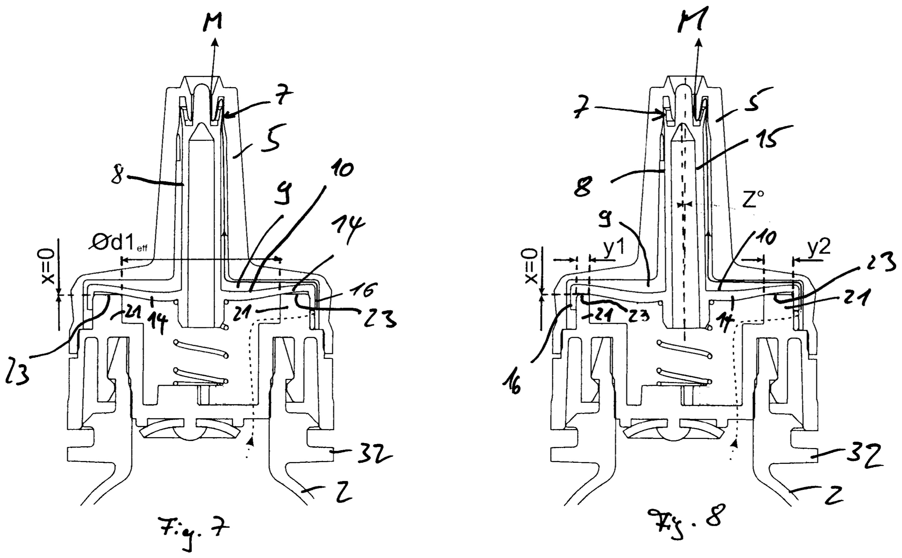

Fig. 7 und Fig. 8 zeigen Ausführungsbeispiele, bei denen der taktil erfassbare Kraftveränderungspunkt längs des Federweges der Ventilfeder 13 variiert werden kann. Die taktile Rückmeldung kann verstärkt werden durch Wahl einer bestimmten Wanddicke y1, y2 des Tellerrandes 21 (Fig. 8). Mit zunehmender Wanddicke verkleinert sich der effektive Durchmesser der Druckbeaufschlagungsfläche 10 und verstärkt damit die Bewegungswiderstandserhöhung, wie Fig. 7 zeigt. Fig. 8 zeigt ferner die Möglichkeit, die Wanddicke des Tellerrandes 21 unsymmetrisch zur axialen Mittelachse der Austragvorrichtung 1 auszubilden. Dies führt zu einem Verkippen des Ventilkörpers 8 zur Austragöffnung 6 mit der Möglichkeit, ein Abscheren eines sich bildenden Tropfens vom Tropfadapter 5 zu erreichen.

Die Ausbildung von Ventilsitz 11 und Dichtkante 12 ist in Fig. 9a, 9b und Fig. 10a, 10b für unterschiedliche Abdichtungen dargestellt. Eine flächige Abdichtung ist in Fig. 9a, 9b dargestellt. Fig. 9a zeigt einen geschlossenen Zustand des Auslassventils 7 mit einer flach-flächigen Dichtkante 12 für eine flächige Abdichtung. Der geöffnete Zustand ist in Fig. 9b gezeigt. Fig. 10a zeigt einen geschlossenen Zustand des Auslassventils 7 mit einer konisch-flächigen Dichtkante 12 für eine konische Abdichtung. Der geöffnete Zustand ist in Fig. 10b gezeigt. Die Anordnung von Ventilsitz 11 und Dichtkante 12 erfolgt vorzugsweise im Spitzenbereich eines bevorzugt vorgesehenen tubusförmigen Fortsatzes des hier als Tropfadapter 5 ausgebildeten Abgabeadapters, wodurch die Eignung zum konservierungsmittelfreien Einsatz weiter verbessert werden kann.

Fig. 11 und Fig. 12 zeigen den Austragkopf 3 mit dem jeweiligen Belüftungsweg D, der gegenüber dem Vorratsbehälter 2 und dessen Innenraum 25 mittels einer Dichtvorrichtung abgedichtet ist.

Gemäß Fig. 11 ist zur Abdichtung eine Dichtscheibe 26 vorgesehen. Die Dichtscheibe 26 ist konkav zum Inneren des Vorratsbehälters 2 gebogen. Die Dichtscheibe 26 liegt an einer Dichtkante 27 benachbart eines Kanals 28 für den Belüftungsweg an. Die Dichtkante 27 bildet quasi einen Ventilsitz einer rückschlagventilartig funktionierenden Dichtscheibe 26.

Gemäß Fig. 12 ist als Dichtvorrichtung ein Dichtstopfen 29 vorgesehen. Der Dichtstopfen 29 weist Dichtlippen 30 auf, die ebenfalls rückschlagventilartig arbeiten, wozu ein Ventilsitz an einer Wandung 31 ausgebildet sein kann.

Kombinierbar ist die erfindungsgemäße Lösung schließlich auch mit aus dem Stand der Technik bekannten Lösungen, die Austragvorrichtung 1 in flüssigkeitsführenden Bereichen mit Bakteriziden, also Bakterien tötenden, oder bakteristatischen, also Bakterienwachstum verhindernden Oberflächen auszugestalten, um gegebenenfalls auftretende Verunreinigungen zu beseitigen oder zu vermeiden.

Als Material für die Membran kann vorgesehen sein Polyethylen (PE), ein thermoplastisches Elastomer (TPE), Polypropylen (PP) oder ein synthetisches Polymer, wie beispielsweise Silikon.

Schließlich kann in bekannter Weise eine Schutzkappe auf dem Austragkopf 3 aufgesetzt werden.

Die Austragvorrichtung 1 kann für jede Art fluiden Mediums verwendet werden. Das Medium kann mit oder ohne Konservierungsstoffe einfüllbar sein. Ferner ist besonders bevorzugt, dass die Höhe und/oder radiale Breite des Tellerrandes 21 und dessen Kopffläche 23 zum Erzeugen von Momenten zur taktilen Rückmeldung der Membranverformung bei Veränderung des Kammervolumens der Ventilvorkammer 9 abhängig von einer Auslenkbewegung der Membran 14 vorgesehen sind. Weiterhin bevorzugt ist, dass eine Bewegungswiderstandserhöhung längs eines Verformungsweges der Membran 14 für eine Tropfenabgabe einstellbar ist. Bevorzugt ist, dass ein taktil erfassbarer Kraftveränderungspunkt längs des Federweges der Ventilfeder 13 variierbar ist. Bevorzugt ist, dass der taktilen Rückmeldung ein anschlagartiger Druck-Hub-Widerstand wie ein Druckpunkt zugeordnet ist. Bevorzugt ist, dass die Momente zur taktilen Rückmeldung der Membranverformung bestimmt sind durch den in Abhängigkeit von der wirkenden Druckkraft variierbaren effektiven Flächeninhalt der Druckbeaufschlagungsfläche 10.

Nachfolgend wird noch eine Funktionsbeschreibung der erfindungsgemäßen Austragvorrichtung 1 gegeben:
Die Austragvorrichtung 1 wird zunächst gefüllt, indem Medium in den Vorratsbehälter 2 abgefüllt wird. Anschließend wird der Austragkopf 3 mit dem Tropfsystem von oben entlang der Längsachse vorzugsweise aufgeschnappt als Verschluss 4. Um die dabei axial wirkenden Kräfte nicht in eine ungewollte oder dauerhaft deformierende Flaschenverformung münden zu lassen, kann die Flasche während des Aufschnappvorgangs unterhalb eines Transferringes 32 (vgl. Fig. 3) abgestützt werden. Durch den Aufschnappvorgang kann der Austragkopf 3 nicht werkzeuglos lösbar mit dem Vorratsbehälter 2 verbunden werden.

Betätigt wird die Austragvorrichtung 1 durch ein Quetschen des Vorratsbehälters 2, wodurch sich dieser elastisch verformen lässt und daraus resultierend das Vorratsbehältervolumen verkleinert wird. Hierdurch bedingt baut sich ein positiver Innendruck auf. Durch den Innendruck wird Medium bzw. Flüssigkeit durch den Flüssigkeitsweg M bewegt. Bei einer Überkopfanwendung der Austragvorrichtung 1 beispielsweise als Augentropfer verläuft dieser Flüssigkeitsweg M dann stromabwärts. Das Medium passiert dabei vorzugsweise eine Engstelle, die Fließbremse 18, im System. Diese Querschnittsverringerung erzeugt einen Energieabbau durch Rückstau und verhindert ein zu schnelles Durchströmen des Medienkanals, wodurch eine Strahlbildung beim Austritt des Mediums wirksam verhindert werden kann. Die Fließbremse 18 kann im Querschnitt an die Medienparameter (wie z.B. Viskosität und Oberflächenspannung) abgestimmt werden. Wird der Querschnitt für das Medium zu klein gewählt, kann die Betätigungskraft bis zur Nicht-Betätigungskraft ansteigen. Durch die eingebrachte Federkraft der Ventilfeder 13 und gegebenenfalls einer Vorspannung der Membran 14 wird das Auslassventil 7 in Ruhelage verschlossen. Das Verschließen erfolgt vorzugsweise nahe der Austragöffnung 6 mikrobiologisch dicht (vgl. Fig. 9a, 9b und Fig. 10a, 10b). Der bei einer Betätigung ansteigende Mediendruck baut über die Druckbeaufschlagungsfläche 10 in der Ventilvorkammer 9 eine Gegenkraft F auf (vgl. Fig. 4), wodurch sich die Membran in Richtung Vorratsbehälter 2 elastisch verformt. Gleichzeitig sorgt der ansteigende Innendruck im Inneren 25 des Vorratsbehälters 2 dafür, dass sich die elastisch verformbare Dichtscheibe 26 selbstverstärkend gegen die Dichtkante 27 eines vorzugsweise zylindrischen Fortsatzes unterhalb der mikrobiologisch wirkenden Filteranordnung 19 anpresst und den Luftweg D so sicher gegen eindringendes Medium schützt (vgl. Fig. 11). Durch die Verformung der Membran 14 bewegt sich der Ventilkörper 8 axial in die gleiche Richtung Y und löst die verschließende Berührung der Dichtfläche 11, 12 bei Erreichen eines Grenzdrucks, dem ein bestimmter Verschiebeweg des Ventilkörpers 8 in Richtung Y aufgrund einer Membranverformung zugeordnet ist. Der entstehende Öffnungsspalt (vgl. Fig. 9b, Fig. 10b) lässt Medium passieren. Sobald sich ausreichend Medium an der Austragöffnung 6 angesammelt hat, tropft dies schwerkraftbedingt ab und kann so appliziert werden.

Nach dem Abtropfen entlastet der Anwender den Vorratsbehälter 2 durch ein Lösen der Betätigungskraft. Dadurch bedingt sinkt der Druck innerhalb des Vorratsbehälters 2 schnell ab. Die Ventilfeder 13 ist dann, gegebenenfalls in Kombination mit einer Vorspannung der Membran 14, in der Lage, die Verformung der Membran 14 in ihre Ausgangslage zurückzudrücken. Über den Ventilköper 8 verschließt sich so die Dichtfläche 11, 12 (vgl. Fig. 9a, Fig. 10) wieder dauerhaft bis zur nächsten Betätigung. Durch das ausgebrachte Medienvolumen in Kombination mit der Rückstellung des Vorratsbehälters 2 und das Abfallen der Betätigungskraft kehren sich die Druckverhältnisse um und es entsteht innerhalb des Vorratsbehälters 2 jetzt ein Unterdruck, welcher das Verschließen der Dichtfläche 11, 12 zusätzlich unterstützt und beschleunigt. Der Unterdruck sorgt ebenfalls dafür, dass sich die elastische Dichtscheibe 26 lösen kann und innerhalb kürzester Zeit ein Nachströmen von mikrobiologisch gefilterter Luft mittels Filter 19 zum Volumenausgleich zugelassen wird. Nach dem Druck- und Volumenausgleich verhindert die mit Vorspannung verbaute Dichtscheibe 26 (vgl. Fig. 11) ein Eindringen von Flüssigkeit in den Luftweg.

Optional kann für die Funktion der Dichtscheibe 26 auch ein Dichtstopfen 29 (vgl. Fig. 12) Anwendung finden, der die Belüftungsfunktion gleichermaßen durch kollabierende Dichtlippen 30 erreicht. Auch hierbei handelt es sich um ein durch Mediendruck selbstverstärkendes System.

Erfindungsgemäß kann die Austragvorrichtung 1 für eine Betätigung derart ausgelegt sein, dass das Ansteigen des Mediendrucks über der Druckbeaufschlagungsfläche 10 in der Ventilvorkammer 9, wodurch eine Gegenkraft F aufbaut und die Membran 14 in Richtung Vorratsbehälter 2 elastisch verformt wird, kombiniert wird mit einem taktil erfassbaren Kraftveränderungspunkt längs des Federweges der Ventilfeder 13. Eine Bewegungswiderstandserhöhung beim Zusammendrücken des Vorratsbehälters 2 zur Tropfenabgabe liefert eine haptisch fühlbare Rückmeldung, die das Erreichen des Grenzdrucks zum Öffnen des Auslassventils 7 als haptisches Ereignis ausbildet. Der Kraft-Weg-Verlauf der Auslenkung der Membran ist variierbar durch eine Reduzierung des effektiven Durchmessers der Druckbeaufschlagungsfläche 10 ausgehend von einem maximalen effektiven Durchmesser, bedingt durch den erfindungsgemäßen Einspannbereich 16. Da sich die Membran 14 vom Umfangsrand her auf den Tellerrand 21 auflegen kann, also benachbart des Einspannbereiches 16, während der Ventilkörper 8 mittig der Membran 14 den aufragenden Ventilstößel 15 aufwärts und abwärts verschiebt, ist der Weg-Kraft-Verlauf im Umfangsrandbereich benachbart dem Einspannbereich 16 kleiner als mittig der Membran 14 mit der Möglichkeit der feinfühligen Einstellung eines Druckpunktes.

Erfolgt eine Membranverformung ohne einen Kontakt mit dem Tellerrand 21, wie in Fig. 5 dargestellt, dann ist der Tellerrand 21 geometrisch so ausgeführt, dass in der Ruhelage und während der gesamten Betätigung (auch bei maximaler Membranverformung durch den Betätigungsinnendruck, also Erreichen des Grenzdrucks) kein Kontakt stattfindet. Während der gesamten Betätigung steht der effektive Membrandurchmesser der Druckbeaufschlagungsfläche zur Verfügung. Der gesamte Flächeninhalt der Druckbeaufschlagungsfläche 10 steht vorzugsweise zur Verfügung, da die Membran 14 nur seitlich im Einspannbereich 16 eine Anlage findet, also im Wesentlichen parallel zur Kraft F (vgl. Fig. 4, Fig. 5).

Eine Membranverformung mit Verkleinerung der Druckbeaufschlagungsfläche durch Anschlagen auf dem Tellerrand 21 ist einstellbar durch Tellerranderhöhung. Die Tellerrandhöhe ist maßlich so ausführbar, dass in Ruhelage kein Kontakt zwischen Tellerrand 21 und Membraninnenseite 24 besteht. Bei Betätigung steht zunächst der gesamte Membrandurchmesser der Druckbeaufschlagungsfläche 10 für die Ventilöffnung zur Verfügung. Gegebenenfalls kann bereits bei minimaler Verformung der Membran 14 diese sich auf den Tellerrand 21 aufsetzen und dadurch der effektive Membrandurchmesser geringfügig verkleinern, wodurch die einwirkende Kraft nunmehr zu gering ist, um eine weitere Verformung der Membran 14 zu erreichen wegen teilweiser Einleitung der Kraft F in den Tellerrand 21 (Fig. 6).

Zusätzlich oder alternativ zu einer Tellerranderhöhung kann die Wanddicke des Tellerrandes 21 erhöht werden. Der Tellerrandinnendurchmesser wird also verkleinert (vgl. Fig. 7). Durch diese Gestaltung ist es möglich, den effektiven Membrandurchmesser um bis zu 70 % im Vergleich zum Ausgangsdurchmesser zu verringern. Dies kann die Kontrollierbarkeit der Tropfenabgabe noch weiter verbessern und kann so ein Überströmen (Strahlbildung) verhindern.

Ein asymmetrisches Ausformen des Tellerrandes 21 (Fig. 8) ermöglicht ein Verkippen des Ventilstößels 15 um einen Winkel (Fig. 8, Z°) und somit ein einseitiges Öffnen des Auslassventils 7, d.h. ein Durchgang für das Medium kann nur partiell freigegeben werden. Die Tellerrandasymmetrie kann bis zum 8-fachen betragen.

## Patentansprüche

1. Austragvorrichtung (1) für ein flüssiges Medium mit einem Vorratsbehälter (2) zur Aufnahme von Medium, einem an dem Vorratsbehälter (2) festlegbaren Austragkopf (3) mit einer Austragöffnung (6) zum Austragen von Medium aus dem Vorratsbehälter (2), einem der Austragöffnung (6) zugeordneten Auslassventil (7) mit einem vorgespannten Ventilkörper (8), der eine Ventilvorkammer (9) durch eine an einer Membran (14) ausgebildete Druckbeaufschlagungsfläche (10) begrenzt, wobei die Druckbeaufschlagungsfläche (10) außenseitig einen lageunveränderlichen Einspannbereich (16) aufweist, und mit einem in den Vorratsbehälter (2) mündenden Druckausgleichskanal (D) mit einer darin eingesetzten mikrobiologisch wirksamen Filteranordnung (19), wobei der Druckausgleichskanal (D) von einem Medienpfad (M) vom Vorratsbehälter (2) über die Ventilvorkammer (9) zur Austragöffnung (6) getrennt ist, **dadurch gekennzeichnet, dass** die Membran (14) zur Ausbildung des lageunveränderlichen Einspannbereiches (16) randseitig an einem Innenbauteil (17) des Austragkopfes (3) durch Laserschweißen fluiddicht befestigt ist, die Druckbeaufschlagungsfläche (10) als Einspannbereich (16) einen kappenartigen Randfortsatz (20) aufweist, der innenseitig auf einen Tellerrand (21) des Innenbauteils (17) des Austragkopfes (3) aufsetzbar ist, und der Tellerrand (21) einen radial nach außen sich erstreckenden Anschlag (22) aufweist, der eine Aufstandsfläche für den Randfortsatz (20) bildet unter Beabstandung der Druckbeaufschlagungsfläche (10) von einer Kopffläche (23) des Tellerrandes (21).

2. Austragvorrichtung (1) nach Anspruch 1, **dadurch gekennzeichnet, dass** der Ventilkörper (8) die Membran (14) derart ausbildet, dass sich von dieser ein Ventilstößel (15) erhebt, der kopfseitig ein Dichtelement (12) zum Verschließen der Austragöffnung (6) trägt.

3. Austragvorrichtung (1) nach einem der Ansprüche 1 bis 2, **dadurch gekennzeichnet, dass** der Ventilkörper (8) mittels einer Ventilfeder (13) in Richtung der Austragöffnung (6) kraftbeaufschlagt ist.

4. Austragvorrichtung (1) nach einem der Ansprüche 1 bis 3, **dadurch gekennzeichnet, dass** der Anschlag (22) einzelne zueinander beabstandet angeordnete Teilanschläge umfasst.

5. Austragvorrichtung (1) nach einem der Ansprüche 1 bis 4, **dadurch gekennzeichnet, dass** der Tellerrand (21) mit der Kopffläche (23) eine flächenhafte Unterlage für eine Abstützung der Membran (14) bei einer Membranverformung unter Mediendruck bildet.

6. Austragvorrichtung (1) nach einem der Ansprüche 1 bis 5, **dadurch gekennzeichnet, dass** der Tellerrand (21) ein um eine axiale Mittelachse der Austragvorrichtung (1) am Innenbauteil (17) angeformter ringförmiger Steg ist, der bezüglich Höhe und/oder Breite variierbar ist.

7. Austragvorrichtung nach Anspruch 6, **dadurch gekennzeichnet, dass** die Höhe des Steges des Tellerrandes (21) derart gewählt ist, dass sich die Membran (14) auf der Kopffläche (23) abstützt vor oder bei Erreichen eines Grenzdrucks in der Ventilkammer (9) durch Verändern des Kammervolumens.

8. Austragvorrichtung nach einem der Ansprüche 1 bis 7, **dadurch gekennzeichnet, dass** über die Höhe und/oder radiale Breite des Tellerrandes (21) und dessen Kopffläche (23) ein effektiver Durchmesser der Druckbeaufschlagungsfläche (10) einstellbar ist.

9. Austragvorrichtung (1) nach einem der Ansprüche 1 bis 8, **dadurch gekennzeichnet, dass** die Höhe und/oder radiale Breite des Tellerrandes (21) und dessen Kopffläche (23) zum Erzeugen von Momenten zur taktilen Rückmeldung der Membranverformung bei Veränderung des Kammervolumens der Ventilvorkammer (9) abhängig von einer Auslenkbewegung der Membran (14) vorgesehen sind.

10. Austragvorrichtung (1) nach Anspruch 9, **dadurch gekennzeichnet, dass** eine Wanddicke des Tellerrandes (21) unsymmetrisch zur axialen Mittelachse der Austragvorrichtung (1) ausgebildet ist.

11. Austragvorrichtung (1) nach Anspruch 9 oder 10 **dadurch gekennzeichnet, dass** eine Bewegungswiderstandserhöhung längs eines Verformungsweges der Membran (14) für eine Tropfenabgabe einstellbar ist.

12. Austragvorrichtung (1) nach einem der Ansprüche 9 bis 11, **dadurch gekennzeichnet, dass** ein taktil erfassbarer Kraftveränderungspunkt längs des Federweges einer Ventilfeder (13) variierbar ist.

13. Austragvorrichtung (1) nach einem der Ansprüche 9 bis 12, **dadurch gekennzeichnet, dass** der taktilen Rückmeldung ein anschlagartiger Druck-Hub-Widerstand wie ein Druckpunkt zugeordnet ist.

14. Austragvorrichtung (1) nach einem der Ansprüche 9 bis 13, **dadurch gekennzeichnet, dass** die Momente zur taktilen Rückmeldung der Membranverformung bestimmt sind durch den in Abhängigkeit von der wirkenden Druckkraft variierbaren effektiven Flächeninhalt der Druckbeaufschlagungsfläche (10).

15. Austragvorrichtung nach einem der Ansprüche 1 bis 14, **dadurch gekennzeichnet, dass** der Druckausgleichskanal (D) gegenüber dem Vorratsbehälter (2) und dessen Innenraum (25) mittels einer Dichtvorrichtung abgedichtet ist.

16. Austragvorrichtung (1) nach Anspruch 15, **dadurch gekennzeichnet, dass** die Dichtvorrichtung als Dichtscheibe (26) oder Dichtstopfen (29) ausgebildet ist.

17. Austragvorrichtung nach einem der Ansprüche 1 bis 16, **dadurch gekennzeichnet, dass** eine Fließbremse (18) vorgesehen ist, über die Medium aus dem Vorratsbehälter (2) in den Mediumpfad (M) drückbar ist.

18. Austragvorrichtung nach einem der Ansprüche 1 bis 17, **dadurch gekennzeichnet, dass** der Austragkopf (3) als Außenbauteil einen Abgabeadapter mit einem tubusförmigen Fortsatz umfasst, der ein Tropfadapter (5) für die Ausgabe von Augentropfen ist.

## Claims

1. A dispenser (1) for a liquid, having a reservoir container (2) for receiving medium, having a discharge head (3) which is able to be mounted to the reservoir container (2) and has a discharge opening (6) for discharging medium from the reservoir container (2), having a discharge valve (7) which is associated with the discharge opening (6) and which has a preloaded valve body (8) which delimits a valve ante chamber (9) by way of a pressure applying surface (10) formed on a diaphragm (14), wherein the pressure applying surface (10) has at the outside a positionally fixed clamping area (16), and having a pressure compensation channel (D) which opens into the reservoir container (2) and has a microbiologically active filter arrangement (19) inserted in said channel, wherein the pressure compensation channel (D) is separated from a media path (M) from the reservoir container (2) via the valve ante chamber (9) to the discharge opening (6), **characterized in that** for the design of the positionally fixed clamping area (16), the diaphragm (14) is, at the boundary side, fastened in a fluid-tight manner by way of laser welding to an inner component (17) of the discharge head (3), and the pressure applying surface (10) has, as a clamping area (16), a cap-like rim projection (20) which is able to be mounted at the inside on an outer rim of a disc (21) of the inner component (17) of the discharge head (3), and the outer rim of the disc (21) has a radially outwardly extending stop (22) which forms a support surface for the rim projection (20) such that the pressure applying surface (10) is spaced apart from an upper surface (23) of the outer rim of the disc (21).

2. Dispenser (1) as claimed in claim 1, **characterized in that** the valve body (8) forms the diaphragm (14) such that a valve plunger (15), bearing at the upper side a sealing element (12) for closing off the discharge opening (6), rises therefrom.

3. Dispenser (1) as claimed in one of claims 1 to 2, **characterized in that**, by means of a valve spring (13), force is applied to the valve body (8) in the direction of the discharge opening (6).

4. Dispenser (1) as claimed in one of claims 1 to 3, **characterized in that** the stop (22) comprises individual sub-parts which are arranged spaced apart from one another.

5. Dispenser (1) as claimed in one of claims 1 to 4, **characterized in that** the outer rim of the disc (21) provides with the head surface (23) a flat base for support of the diaphragm (14) during diaphragm deformation under media pressure.

6. Dispenser (1) as claimed in one of claims 1 to 5, **characterized in that** the outer rim of the disc (21) is a ring-like bar which is integrally formed on the inner component (17) about an axial central axis of the discharge device (1) and which is able to be varied with respect to height and/or width.

7. Dispenser as claimed in claim 6, **characterized in that** the height of the bar of the outer rim of the disc (21) is selected such that, prior to or upon reaching a pressure limit in the valve chamber (9), the diaphragm (14) is supported on the upper surface (23) as a result of the chamber volume being changed.

8. Dispenser as claimed in one of claims 1 to 7, **characterized in that** an effective diameter of the pressure applying surface (10) is settable via the height and/or radial width of the outer rim of the disc (21) and of the upper surface (23) thereof.

9. Dispenser (1) as claimed in one of claims 1 to 8, **characterized in that** the height and/or radial width of the outer rim of the disc (21) and of the upper surface thereof (23) are provided for the generation of moments for the tactile feedback of the diaphragm deformation when the chamber volume of the valve ante chamber (9) is changed in a manner dependent on a deflection movement of the diaphragm (14).

10. Dispenser (1) as claimed in claim 9, **characterized in that** a wall thickness of the outer rim of the disc (21) is provided in an asymmetrical manner with respect to the axial central axis of the dispenser (1).

11. Dispenser (1) as claimed in claim 9 or 10, **characterized in that** an increase in movement resistance along a deformation path of the diaphragm (14) is settable for drop dispensing.

12. Dispenser (1) as claimed in one of claims 9 to 11, **characterized in that** a force change point, which is detectable in a tactile manner, is able to be varied along the spring travel of a valve spring (13).

13. Dispenser (1) as claimed in one of claims 9 to 12, **characterized in that** a stop-type pressure-displacement resistance, such as a pressure point, is associated with the tactile feedback.

14. Dispenser (1) as claimed in one of claims 9 to 13, **characterized in that** the moments for the tactile feedback of the diaphragm deformation are determined by the effective surface area of the pressure applying surface (10), which surface area is able to be varied in a manner dependent on the active pressure force.

15. Dispenser as claimed in one of claims 1 to 14, **characterized in that** the pressure equalization channel (D) is sealed off with respect to the reservoir container (2) and the inner space (25) thereof by means of a sealing device.

16. Dispenser (1) as claimed in claim 15, **characterized in that** the sealing device is designed as a sealing disk (26) or sealing plug (29).

17. Dispenser as claimed in one of claims 1 to 16, **characterized in that** provision is made of a flow reducer (18) via which medium is able to be forced into the medium path (M) from the reservoir container (2).

18. Dispenser as claimed in one of claims 1 to 17, **characterized in that** the discharge head (3) comprises as an outer component a dispensing adapter which has a tubular extension and which is a drop adapter (5) for the dispensing eye drops.

## Revendications

1. Dispositif de décharge (1) pour un fluide liquide avec un réservoir (2) pour la réception de fluide, une tête de décharge (3) pouvant être fixée au réservoir (2) avec une ouverture de décharge (6) pour la décharge de fluide du réservoir (2), une soupape de sortie (7) associée à l'ouverture de décharge (6) avec un corps de soupape (8) précontraint qui délimite une préchambre de soupape (9) par une surface de sollicitation de pression (10) réalisée au niveau d'une membrane (14), dans lequel la surface de sollicitation de pression (10) présente côté extérieur une zone de serrage (16) à position non modifiable, et avec un canal de compensation de pression (D) débouchant dans le réservoir (2) avec un agencement de filtre (19) actif microbiologiquement inséré dedans, dans lequel le canal de compensation de pression (D) est séparé d'un trajet de fluide (M) du réservoir (2) par la chambre de soupape (9) à l'ouverture de décharge (6), **caractérisé en ce que** la membrane (14) est fixée de manière étanche au fluide pour la réalisation de la zone de serrage (16) à position non modifiable côté bord au niveau d'un composant intérieur (17) de la tête de décharge (3) par soudage au laser, et la surface de sollicitation de pression (10) présente comme zone de serrage (16) un prolongement de bord (20) de type capuchon qui peut être placé côté intérieur sur un bord de disque (21) du composant intérieur (17) de la tête de décharge (3), et le bord de disque (21) présente une butée (22) s'étendant radialement vers l'extérieur qui forme une surface de contact pour le prolongement de bord (20) en espaçant la surface de sollicitation de pression (10) d'une surface de tête (23) du bord de disque (21).

2. Dispositif de décharge (1) selon la revendication 1, **caractérisé en ce que** le corps de soupape (8) réalise la membrane (14) de telle manière qu'un poussoir de soupape (15) se relève de celle-ci, qui porte côté tête un élément étanche (12) pour la fermeture de l'ouverture de décharge (6).

3. Dispositif de décharge (1) selon l'une des revendications 1 à 2, **caractérisé en ce que** le corps de soupape (8) est sollicité à force au moyen d'un ressort de soupape (13) en direction de l'ouverture de décharge (6).

4. Dispositif de décharge (1) selon l'une des revendications 1 à 3, **caractérisé en ce que** la butée (22) comporte des butées partielles individuelles agencées à distance les unes des autres.

5. Dispositif de décharge (1) selon l'une des revendications 1 à 4, **caractérisé en ce que** le bord de disque (21) avec la surface de tête (23) forme un support de surface pour un appui de la membrane (14) lors d'une déformation de membrane sous pression de fluide.

6. Dispositif de décharge (1) selon l'une des revendications 1 à 5, **caractérisé en ce que** le bord de disque (21) est une nervure annulaire formée autour d'un axe médian axial du dispositif de décharge (1) au niveau du composant intérieur (17) qui est variable en hauteur et/ou largeur.

7. Dispositif de décharge selon la revendication 6, **caractérisé en ce que** la hauteur de la nervure du bord de disque (21) est choisie de telle manière que la membrane (14) s'appuie sur la surface de tête (23) avant ou lors de l'atteinte d'une pression limite dans la chambre de soupape (9) par modification du volume de chambre.

8. Dispositif de décharge (1) selon l'une des revendications 1 à 7, **caractérisé en ce qu'**un diamètre effectif de la surface de sollicitation de pression (10) peut être réglé par la hauteur et/ou la largeur radiale du bord de disque (21) et sa surface de tête (23).

9. Dispositif de décharge (1) selon l'une des revendications 1 à 8, **caractérisé en ce que** la hauteur et/ou la largeur radiale du bord de disque (21) et sa surface de tête (23) sont prévues pour la génération de couples pour le retour tactile de la déformation de membrane tors de la modification du volume de chambre de la préchambre de soupape (9) en fonction d'un mouvement de déviation de la membrane (14).

10. Dispositif de décharge (1) selon la revendication 9, **caractérisé en ce qu'**une épaisseur de paroi du bord de disque (21) est réalisée de manière non symétrique à l'axe médian axial du dispositif de décharge (1).

11. Dispositif de décharge (1) selon la revendication 9 ou 10, **caractérisé en ce qu'**une augmentation de résistance de mouvement est réglable le long d'une voie de déformation de la membrane (14) pour une éjection de gouttes.

12. Dispositif de décharge (1) selon l'une des revendications 9 à 11, **caractérisé en ce qu'**un point de modification de force pouvant être détecté de manière tactile est variable le long de la course de ressort d'un ressort de soupape (13).

13. Dispositif de décharge (1) selon l'une des revendications 9 à 12, **caractérisé en ce qu'**au retour tactile est associée une résistance de pression et de levage de type butée telle qu'un point de pression.

14. Dispositif de décharge (1) selon l'une des revendications 9 à 13, **caractérisé en ce que** les couples sont déterminés pour le retour tactile de la déformation de membrane par la superficie effective variable en fonction de la force de pression active de la surface de sollicitation de pression (10).

15. Dispositif de décharge selon l'une des revendications 1 à 14, **caractérisé en ce que** le canal de compensation de pression (D) est rendu étanche par rapport au réservoir (2) et son espace intérieur (25) au moyen d'un dispositif étanche.

16. Dispositif de décharge (1) selon la revendication 15, **caractérisé en ce que** le dispositif étanche est réalisé comme disque étanche (26) ou bouchon étanche (29).

17. Dispositif de décharge selon l'une des revendications 1 à 16, **caractérisé en ce qu'**un frein d'écoulement (18) est prévu, par lequel du fluide peut être pressé du réservoir (2) dans le trajet de fluide (M).

18. Dispositif de décharge selon l'une des revendications 1 à 17, **caractérisé en ce que** la tête de décharge (3) comporte comme composant extérieur un adaptateur d'éjection avec un prolongement en forme de tube qui est un adaptateur de goutte (5) pour la sortie de gouttes ophtalmologiques.
